# DEMANDE DE BREVET EUROPEEN

(11) **EP 0 818 531 A2**
(43) Date de publication de la demande: **14.01.1998**
(21) Numéro de dépôt: 97401547.1
(22) Date de dépôt: 01.07.1997
(51) Int. Cl.: C12N 9/24, C12N 1/20

(54) **Procédé de préparation de K-carraghénase**

(30) Priorité: 09.07.1996 FR 9608530
(71) Demandeur: CECA S.A., 92800 Puteaux (FR)
(72) Inventeur: Gancet, Christian, 64140 Lons (FR); Remaud-Simeon, Magali, 31520 Ramonville-Saint-Agne (FR); Willemot, René-Marc, 31450 Pompertuzat (FR)
(74) Mandataire: Haicour, Philippe

(57) **Abrégé**

L'invention concerne un procédé microbiologique de préparation de K-carra-ghénase qui consiste à contrôler le pH de la culture au moyen d'une base azotée assimilable par la bactérie.

Le microorganisme est choisi parmi le groupe constitué par *Pseudomonas carrageenovora, Alteromonas et Cytophaga.*

## Description

La présente invention concerne un procédé microbiologique de préparation de K-carraghénase.

Les K-carraghénases sont des enzymes d'hydrolyse des K-carraghénanes, polysaccharides sulfatés constitutifs de la paroi des algues rouges.

Les K-carraghénases sont généralement produites par des bactéries marines, par exemple les *Pseudomonas carrageenovora* (WEIGL J. and YAPHE W., Canadian Journal of Microbiology, vol.12, pp. 939-947, 1966 ; Mc LEAN M.W. and WILLIAMSON F.B., Eur. J. Biochem., vol.93, pp. 553-558, 1979). Exocellulaires et spécifiques des liaisons β-1,4, ces enzymes permettent d'obtenir des oligosaccharides sulfatés, notamment le néocarrabiose-4-O-sulfate qu'il n'est pas possible de préparer par traitement acide du K-carraghénane (perte du groupement sulfate).

ØSTGAARD K. et al. (Enzyme Microb. Technol., vol.15, pp. 326-333, 1993) ont montré que les K-carraghénases peuvent être produites à partir d'une culture bactérienne réalisée dans un fermenteur. L'activité enzymatique obtenue, de l'ordre de 5 unités galactose par millilitre (U gal/ml), reste malgré tout faible ce qui impose d'augmenter le nombre des fermentations lorsqu'on souhaite des quantités importantes d'enzymes. En outre, on constate que si la production de biomasse est reproductible, l'activité enzymatique quant à elle a tendance à varier dans une large mesure.

Il a maintenant été trouvé qu'en régulant le pH de la culture au moyen d'une base azotée assimilable par la bactérie, on peut améliorer sensiblement, et de manière reproductible, la production de K-carraghénase.

Plus précisément, le procédé selon l'invention permet d'obtenir une activité enzymatique supérieure à 20 Ugal/ml, et avantageusement comprise entre 40 et 60 Ugal/ml.

Le procédé selon l'invention sera mieux compris à la lumière de la description ci-après.

Dans la mise en oeuvre du procédé selon l'invention, on utilise généralement un fermenteur conventionnel du type batch muni d'une sonde à oxygène dissous, de moyens d'agitation et d'un système de régulation de la température et du pH.

Le volume total du fermenteur n'est pas critique et peut varier dans une large mesure, par exemple de 2 à 100 litres, voire davantage.

En conditions de fonctionnement, on inocule le milieu de culture contenu dans le fermenteur au moyen d'une préculture bactérienne.

La bactérie est généralement choisie parmi le groupe constitué par *Pseudomonas carrageenovora, Alteromonas et Cytophaga.* De préférence, on utilise *Pseudomonas carrageenovora* ATCC 43555 ou NCMB 302.

La préculture bactérienne est généralement obtenue par ensemencement d'un milieu de culture de type W.Y. medium ou New Standard tel que décrit par ØSTGAARD K. et al. (Enzyme Microb. Technol., vol.15, pp. 326-333, 1993) avec la bactérie considérée.

La préculture est généralement réalisée dans une fiole agitée à une température variant de 20 à 30°C, de préférence 24 à 26°C et pendant une durée n'excédant pas 24 heures, de préférence 10 à 20 heures.

La densité optique de la préculture mesurée à 600 nm, varie généralement de 0,5 à 2,5, et de préférence 1 à 2.

En général, le taux d'inoculation du fermenteur au moyen de la préculture est compris entre 1 et 10 % en volume et de préférence 4 et 6 %.

Le milieu de culture dans le fermenteur est généralement choisi parmi les milieux W.Y. médium et New Standard définis ci-avant. De préférence, on utilise le milieu New Standard.

Le pH du milieu de culture est généralement maintenu à 7 ± 1, et de préférence 7 ± 0,5 au moyen d'une base azotée.

La température de la culture est généralement comprise dans les limites indiquées ci-avant pour la préculture.

La base azotée est généralement choisie parmi les composés basiques contenant de l'azote assimilable par la bactérie, tels que l'ammoniaque, les amines aliphatiques, par exemple la méthylamine et l'éthylamine, aromatiques, par exemple la pyridine et la benzylamine, ou hydroxylées, par exemple l'hydroxylamine. De préférence on utilise l'ammoniaque.

Après un temps de culture pouvant varier de 25 à 120 heures, la culture est centrifugée. Le surnageant contient plus de 20 Ugal/ml de K-carraghénase.

L'enzyme peut éventuellement être purifiée par des méthodes conventionnelles, par exemple par dialyse, chromatographie par échange d'ions, précipitation par le sulfate d'ammonium ou ultrafiltration.

Les exemples qui suivent permettent d'illustrer l'invention.

Dans les exemples, on utilise les méthodes ci-après.

La densité optique de la culture est mesurée à 600 nm (DO₆₀₀).

La concentration en lactose (g/l) dans le milieu de culture est mesurée selon la méthode à l'acide dinitrosalicilique décrite par G.I. MILLER (Anal. Chem. vol.31, pages 426-428, 1959).

L'activité K-carraghénase est mesurée selon la méthode décrite ci-avant pour la détermination de la concentration en lactose.

On exprime les résultats en unités galactose par ml de culture (Ugal/ml), une unité galactose correspondant à la quantité d'enzyme qui catalyse la libération d'une micromole de sucre réducteur (équivalent galactose) en une minute.

### EXEMPLE 1

### a) Préparation de l'inoculum

*Pseudomonas carrageenovora* ATCC 43555 est cultivé à 25°C dans une fiole agitée contenant le milieu New Standard selon ØSTGAARD de composition suivante (en g/l) :

| | |
|---|---|
| Extrait de levure | 0,5 |
| Lactose | 20 |
| (NH₄)₂SO₄ | 13 |
| MgSO₄.7H₂O | 0,5 |
| CaCl₂.2H₂O | 0,2 |
| KCl | 0,6 |
| NaCl | 20 |
| K-carraghénane | 1,5 |
| Na₂HPO₄.2H₂O | 2,0 |
| FeSO₄.7H₂O | 0,001 |

Après 24 heures d'incubation, on obtient une culture bactérienne présentant une DO₆₀₀ d'environ 2.

### b) Culture en fermenteur

Dans un fermenteur de 2,5 litres équipé d'une sonde à oxygène dissous, d'un système d'agitation, de moyens de régulation du pH et d'une enveloppe thermostatée, on introduit 1,5 litre du milieu de culture New Standard précité (préalablement stérilisé dans le fermenteur) et 75 ml de la préculture obtenue à l'étape précédente.

Dans le fermenteur, la température est maintenue à 25°C, le pH est régulé à 7 ± 1 au moyen d'une solution ammoniacale à 14 % en poids et l'agitation est égale à 650 RPM.

Les résultats sont présentés dans le tableau 1 suivant.

| **TEMPS (heures)** | **DO**_{**600**} | **O**_{**2**} **(%)** | **pH** | **LACTOSE (g/l)** | **ACTIVITE (Ugal/ml)** |
|---|---|---|---|---|---|
| 0 | 0,010 | 99,8 | 7,10 | 20,8 | 0 |
| 10 | 0,012 | 93,3 | 7,20 | 20,9 | 0 |
| 15 | 0,022 | 75,0 | 7,19 | 19,5 | 0 |
| 20,5 | 0,042 | 68,0 | 7,10 | 19,2 | 1,5 |
| 30 | 0,146 | 50,0 | 6,98 | 18,75 | 6,5 |
| 38 | 0,191 | 47,4 | 6,97 | 16,06 | 9,1 |
| 54 | 0,645 | 20,0 | 6,98 | 6,05 | 27,9 |
| 59 | 0,906 | 36,0 | 6,98 | 1,59 | 27,9 |
| 63 | 0,950 | 56,0 | 6,98 | 0,21 | 27,8 |
| 64,5 | 0,965 | 65,0 | 7,20 | 0,17 | 27,6 |
| 78 | 0,650 | 86,0 | 7,60 | 0,18 | 28,4 |

### EXEMPLE 2

On procède dans les conditions de l'exemple 1 modifiées en ce que l'on utilise *Pseudomonas carrageenovora* NCMB 302.

Les résultats sont présentés dans le tableau 2 suivant.

| **TEMPS (heures)** | **DO**_{**600**} | **O**_{**2**} **(%)** | **LACTOSE (g/l)** | **ACTIVITE (Ugal/ml)** |
|---|---|---|---|---|
| 0 | 0,010 | 94,0 | 20,80 | 0 |
| 14 | 0,061 | - | 20,10 | 4 |
| 17,5 | 0,096 | 67,0 | 19,50 | 9,25 |
| 25 | 0,200 | 50,0 | 18,24 | 18,20 |
| 38 | 0,420 | 50,0 | 11,83 | 47,20 |
| 42 | 0,498 | 41,0 | 9,40 | 47,00 |
| 46,7 | 0,698 | 36,0 | 3,66 | 46,90 |
| 51 | 0,840 | 52,0 | 2,80 | 45,00 |
| 62,5 | 0,780 | 84,0 | 0,15 | 39,60 |
| 66 | 0,530 | 85,0 | 0,21 | 40,30 |
| 69 | 0,490 | - | 0 | 40,50 |

### EXEMPLE 3 (Comparatif)

On procède dans les conditions de l'exemple 1 modifiées en ce que d'une part le pH est régulé au moyen d'une solution de soude 6 N.

Les résultats sont présentés dans le tableau 3 suivant.

| **TEMPS (heures)** | **DO**_{**600**} | **O**_{**2**} **(%)** | **pH** | **LACTOSE (g/l)** | **ACTIVITE (Ugal/ml)** |
|---|---|---|---|---|---|
| 0 | 0,14 | 99,4 | 7,24 | 23,6 | 0 |
| 6 | 0,48 | 69 | 7,07 | 23 | 0 |
| 13 | 1,04 | 70,2 | 7 | 19,30 | 4 |
| 21,75 | 2,90 | 42,6 | 6,99 | 18,30 | 6,13 |
| 27 | 6,37 | 39,2 | 7,05 | 13,50 | 5,33 |
| 31 | 8,58 | 41,8 | 7,08 | 8,50 | 6,50 |
| 45,75 | 16,70 | 53,3 | 7,07 | 2,48 | 5,60 |
| 51,75 | 14,56 | 71,7 | 7,07 | 0,55 | 5,42 |
| 69,75 | 13,26 | 91 | 7,40 | 0,40 | 5,15 |
| 71,50 | 13,44 | 99 | 7,40 | 0,30 | 5,41 |

## Revendications

1. Procédé microbiologique de préparation de K-carraghénase à partir de K-carraghénane en présence de *Pseudomonas carrageenovora, Alteromonas* ou *Cytophaga* qui consiste à réguler le pH de la culture au moyen d'une base azotée assimilable par la bactérie.

2. Procédé selon la revendication 1, caractérisé en ce que la base est choisie parmi l'ammoniaque, les amines aliphatiques, aromatiques ou hydroxylées.

3. Procédé selon la revendication 2, caractérisé en ce que la base est l'ammoniaque.

4. Procédé selon l'une des revendications 1 à 3, caractérisé en ce que l'on utilise *Pseudomonas carrageenovora* ATCC 43555 ou NCMB 302.

5. Procédé selon l'une des revendications 1 à 4, caractérisé en ce que l'activité enzymatique est supérieure à 20 Ugal/ml.
